# EUROPEAN PATENT APPLICATION

(11) **EP 2 567 683 A1**
(43) Date of publication of application: **13.03.2013**
(21) Application number: 12183035.0
(22) Date of filing: 05.09.2012
(51) Int. Cl.: A61F 13/36, A61F 13/44, A61B 5/06, A61B 19/02

(54) **A radiologically visible, surgial gauze and a method for its manufacture**

(30) Priority: 06.09.2011 ES 201131460
(71) Applicant: BC Nonwovens, S.L., 08036 Barcelona (ES)
(72) Inventor: Viñas Pich, Carlos, 08036 Barcelona (ES)
(74) Representative: Barroso Sánchez-Lafuente, Ignacio M.

(57) **Abstract**

A gauze comprising at least two superimposed non-woven fabric webs (1) and at least one radiologically visible yarn (2) arranged between the two non-woven fabric webs (1), the webs (1) and the radiologically visible yarn (2) being bonded to each other. The radiologically visible yarn (2) is of polypropylene compounded with up to 65% by weight of barium sulphate. The method of manufacture of the gauze comprises: - the superimposed arrangement of at least two non-woven fabric webs (1); - the arrangement of at least one radiologically visible yarn (2) between the two superimposed non-woven fabric webs (1); and - the bonding of the non-woven fabric webs (1) and the radiologically visible yarn (2) arranged between said webs (1).

## Description

### Object of the invention

The present invention relates to a radiologically visible, surgical gauze and to a method for its manufacture; said gauze being of the type of those being used in surgical interventions and being radiologically visible in order to allow their detection by means of a radiography and their removal from the patient's body before finishing the intervention.

### Application field of the invention

This gauze and the obtainment method are applicable in the sanitary material sector, and more concretely in the sector of the manufacture of sterile dressings and gauzes for surgical use.

### Background of the invention

In surgical interventions it is common to use sterile gauzes in order to soak them with the blood and to thus facilitate the task of the sanitary personnel. Before closing the wound it is necessary to remove these gauzes since if they are left inside the patient's body they can cause problems with infections and risks for the patient's health. These blood-soaked gauzes are nevertheless difficult to distinguish from the surrounding tissues and organs.

Techniques are already known at present which aim at making these gauzes radiologically visible and at making it possible to detect them in a radiography taken from the patient before closing the wound or the intervention.

For example in the Spanish patent ES497130 for "A process for the obtainment of a radiologically opaque yarn" the existence in the market of yarns coated with X-ray opaque matters is already considered as known, but with the drawback of the impossibility to have them woven into the weft of the gauze since the coating determines a rough and easily strippable surface coat.

Said patent also specifies a technique having been used before in order to solve the same problem and consisting in superimposing on the outer surface of the fabric forming the gauze the aforementioned yarns coated with X-ray opaque matters. This fixation of the radiologically visible yarns to the outside of the gauze is carried out by means of pressure and heat fixation or by means of an adhesive strip, the detachment of the yarns in question possibly taking place if this fixation is not properly carried out. This fixation is besides time-consuming and slows down the production of the gauze since the application of the radiologically visible yarn is carried out on the previously made up gauze.

The solution proposed in the aforementioned Spanish patent ES497130 consists in a process for the obtainment of a radiologically opaque yarn that is in a position to be woven with conventional textile machines and to be incorporated into the weft or warp of the gauze thus precluding the possibility of its being stripped off and providing some reliability in the scanning with X-rays.

An also known document is the Spanish utility model ES1045580U for "A gauze for surgical interventions" where a gauze for surgical interventions is described which is made up of an actual gauze of any conventional type incorporating at least a metallic filament being detectable through electromagnetic means and/or through X-rays.

This document provides for the filament in question to be properly fixed to the base body of the gauze, such as for example by being interlocked in the network of this latter after the obtainment of said base body. The radiologically visible yarn is fixed by means of sewing, an adhesive strip, heat welding or any other conventional locking means within the general context of the gauze. The main problem with this technique is the reduced manufacturing speed. The radiologically visible yarn can besides be stripped off from the surface of the base body if the fixation has not been carried out in a completely adequate manner, the gauze being thus deprived from the feature of radiologic detection.

The European patent EP0272901 for "A surgical fabric with a printed X-ray marker" (Johnson & Johnson Medical) having been validated in Spain with the publication number ES2035868 relates to a method for the preparation of a surgical sponge that comprises an absorbing fabric with an X-ray detectable marker associated to it, said method consisting in applying a fluid radio-opaque polymer composition to the surface of the aforementioned fabric thus forming a visually distinctive pattern, and solidly adhering said polymer composition to said fabric.

The Spanish patent document ES494396 for "A process for obtaining a Roentgen-ray opaque yarn" is known which aims at incorporating a radiologically opaque yarn in a variable number into the weft or warp of a cotton fabric being used to form the gauzes for clinical use. This process is essentially **characterised in that** it consists in incorporating barium into a volume of viscose in a proportion of between 45 and 75 %, said blend being then submitted to extrusion and ulterior twisting in order to thus form the yarn.

The techniques being used to incorporate radiologically visible yarns into gauzes of warp and weft fabric are hence not applicable to the non-woven fabric gauzes, and the surface application of the radiologically visible yarn to non-woven fabric gauzes has the abovementioned drawbacks of a slowing down of the production process and a risk of detachment of the yarn in question.

### Description of the invention

The radiologically visible, surgical gauze being the object of this invention has technical particularities aiming at offering a surer detection of the gauze by means of X-rays and at preventing the separation or detachment of the radiologically visible yarn from the gauze; a method being additionally used for its manufacture which allows to incorporate the radiologically visible yarn during the forming of the gauze, this allowing a quick manufacture without a significant cost increase.

The radiologically visible, surgical gauze of this invention is of the type of those being formed in a non-woven fabric and according to the invention comprises at least two superimposed non-woven fabric webs and at least one radiologically visible yarn arranged between the two non-woven fabric webs, the webs and the radiologically visible yarn being bonded to each other.

In this way the radiologically visible yarn is in the inside of the body of the gauze, between two non-woven fabric webs, this guaranteeing that said yarn won't possibly be detached by accident and that the gauze will be detectable by means of X-rays.

In a preferential embodiment the radiologically visible yarn is of polypropylene compounded with 65% by weight of barium sulphate that is incorporated before the spinning. This yarn has the advantage that the barium sulphate is not detached during the manufacture of the gauze.

The method of manufacture of this radiologically visible, surgical gauze comprises: - the superimposed arrangement of at least two non-woven fabric webs, - the placing of at least one radiologically visible yarn between two of the superimposed fabric webs, and - the bonding of the non-woven fabric webs and the radiologically visible yarn between them.

In one embodiment the superimposed arrangement of the non-woven fabric webs and the radiologically visible yarn between said webs is carried out continuously. The supply of radiologically visible yarn or yarns is carried out from at least one dispenser spool, whereas the supply of the non-woven fabric webs can be carried out: either from respective dispenser bobbins, or directly from the machine being used for the manufacture of said non-woven fabric webs.

This method allows to manufacture the gauze in a continuous manner and thus allows to attain a high manufacturing speed with no significant cost as regards the incorporation of the radiologically visible yarn.

In an embodiment variant the non-woven fabric webs between which the radiologically visible yarn is arranged can be directly supplied from the machine being used for the manufacture of said webs.

The bonding of the non-woven fabric webs with the radiologically visible yarn arranged between them can be exemplarily carried out by means of punching and embossing techniques, and preferably by projecting pressurised water jets through the assembly being formed by said superimposed webs and the radiologically visible yarn and against a micropierced drum supporting said assembly. This technique for bonding the webs with the radiologically visible yarn in their inside is also known as "spunlace" technique or hydrobonding of carded fibres.

The through-flow of the pressurised water causes the elements to be bonded together with enough cohesion as to prevent their mutual detachment and with no risk of breakage of the web fibres or of the radiologically visible yarn; the webs maintaining all their initial mechanical strength.

In another alternative embodiment the bonding means can comprise a needle punching operation (a technique that is also known as "needlepunch", or mechanical punching) which brings about a quick interlacing of the webs.

### Description of the figures

In order to complement the description being made and for the purpose of facilitating the understanding of the characterising features of the invention the present specification is being accompanied by a set of drawings wherein the following has been represented with an illustrative and nonlimiting character:
- Figure 1 shows a perspective view of a gauze wherein the webs have been separated in order to observe the two radiologically visible yarns.
- Figure 2 shows a diagrammatic view of a method for the manufacture of the surgical gauze as per the invention.

### Preferential embodiment of the invention

As can be seen in the aforementioned figures the gauze being the object of this invention comprises two superimposed webs (1) of non-woven fabric, and radiologically visible yarns (2) being longitudinally arranged between both webs (1).

The webs (1) are in this case carded webs (non-woven fabric) being made of a blend of viscose and polyester fibres in a by-weight ratio of 70/30, and the radiologically visible yarns (2) are made in polypropylene with 65% by weight of barium sulphate that is introduced before spinning them.

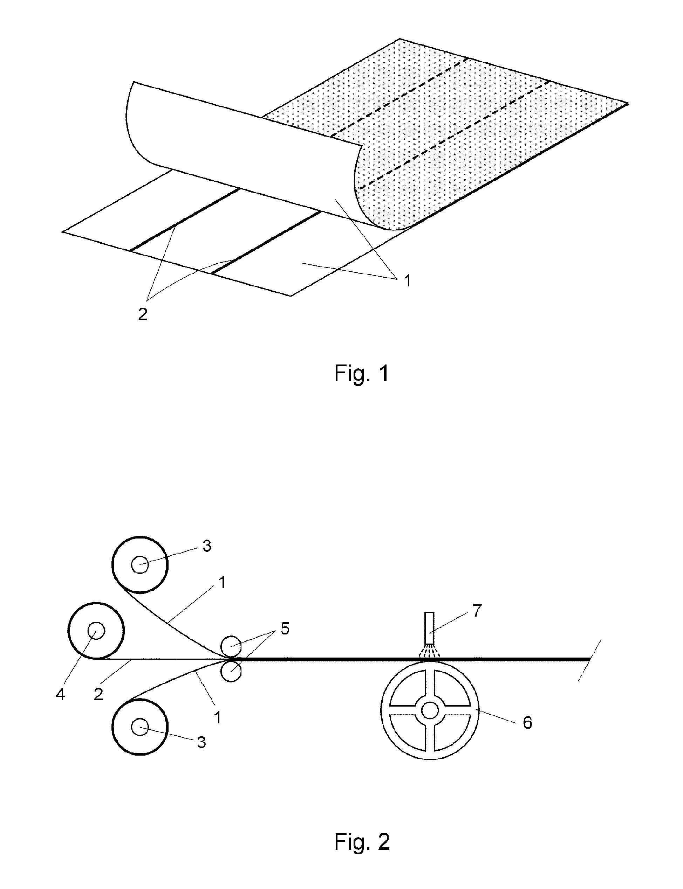

In figure 2 the carded webs (1) of non-woven fabric are superimposedly supplied by bobbins (3) and the radiologically visible yarns (2) are supplied from dispenser spools (4) and are thus placed between the webs (1).

The two webs (1) and the radiologically visible yarns (2) are sent to joining rolls (5), or in another case are deposited on an apron, before passing through the means being used to bond the webs (1) and the radiologically visible yarns (2). In the case being shown the bonding means use a "spunlace" or hydrobonding technology and comprise injectors (7) projecting high pressure water jets through the assembly being formed by the non-woven fabric webs (1) and the radiologically visible yarns (2) and against the micropierced drum (6) supporting the gauze assembly, said water jets bringing about the bonding of the different layers without causing their breakage.

The manufactured gauze is passed to a drying station and is afterwards wound for its storage, or for passing it to a cutting station to be thus cut to the required dimension and to thereupon be sterilised and packaged.

Once having sufficiently described the nature of the invention, as well as a preferential exemplary embodiment, it is hereby stated for the record and for all relevant purposes that the materials, shape, size and arrangement of the described elements can be modified, provided that this does not involve an alteration of the essential features of the invention which are claimed below.

## Claims

1. A radiologically visible, surgical gauze of the type of those being formed in a non-woven fabric, **characterised in that** it comprises at least two superimposed non-woven fabric webs (1) and at least one radiologically visible yarn (2) arranged between the two non-woven fabric webs (1), the webs (1) and the radiologically visible yarn (2) being bonded to each other.

2. A gauze as per claim 1, **characterised in that** the radiologically visible yarn (2) is of polypropylene compounded with up to 65% by weight of barium sulphate.

3. A method of manufacture of the gauze of the foregoing claims, **characterised in that** it comprises: - the superimposed arrangement of at least two non-woven fabric webs (1), - the arrangement of at least one radiologically visible yarn (2) between two of the superimposed non-woven fabric webs (1), and - the bonding of the non-woven fabric webs (1) and the radiologically visible yarn (2) arranged between said webs (1).

4. A method as per claim 3, **characterised in that** the superimposed arrangement of the non-woven fabric webs (1) and the radiologically visible yarn (2) between said webs (1) is carried out continuously.

5. A method as per claim 4, **characterised in that** the continuous supply of the radiologically visible yarn or yarns (2) is carried out from at least one dispenser spool (4).

6. A method as per any of claims 4 to 5, **characterised in that** the continuous supply of non-woven fabric webs (1) is carried out from respective dispenser bobbins (3).

7. A method as per any of claims 4 to 5, **characterised in that** the continuous supply of non-woven fabric webs (1) is directly carried out from machines being used for the manufacture of said non-woven fabric webs (1).

8. A method as per claim 4, **characterised in that** the bonding of the webs (1) and the radiologically visible yarn (2) comprises a step of projecting pressurised water jets through the assembly being formed by said superimposed webs (1) and the radiologically visible yarn (2) and against a micropierced drum (6) supporting said assembly.
